(19) Europäisches Patentamt
European Patent Office
Office européen des brevets

(11) Veröffentlichungsnummer: **0 508 296 B1**

(12) **EUROPÄISCHE PATENTSCHRIFT**

(45) Veröffentlichungstag der Patentschrift: **22.11.95**

(51) Int. Cl.[6]: **C07F 9/30**, C07F 9/38, C07C 229/20

(21) Anmeldenummer: **92105615.6**

(22) Anmeldetag: **01.04.92**

# (54) Verfahren zur Herstellung phosphorhaltiger L-Aminosäuren, deren Derivate und Zwischenprodukte für dieses Verfahren.

(30) Priorität: **06.04.91 DE 4111188**

(43) Veröffentlichungstag der Anmeldung:
**14.10.92 Patentblatt 92/42**

(45) Bekanntmachung des Hinweises auf die Patenterteilung:
**22.11.95 Patentblatt 95/47**

(84) Benannte Vertragsstaaten:
**AT BE CH DE FR GB IT LI NL**

(56) Entgegenhaltungen:
**DE-A- 2 856 260**

**CHEMICAL ABSTRACTS (STN online), American Chemical Society, CA 80(21): 120 734r**

**JOURNAL OF THE AMERICAN CHEMICAL SOCIETY 1990, Band 112, American Chemical-Society G.M. SALITURU & C.A. TOWNSEND "Total Syntheses of (-)Nocardicins A-G: A Biogenetic Approach"**

**BULLETIN OF THE CHEMICAL SOCIETY OF JAPAN, Band 40, Nr. 9, 1967, T. CHEN"Azetidines II. Some Functional Derivatives of Azetidines" Seiten 2398-2401**

**Tetrahedron Band 44(1988),No. 17,Seiten 5479-5486**

(73) Patentinhaber: **Hoechst Schering AgrEvo GmbH**
**Gerichtstrasse 27**
**D-13342 Berlin (DE)**

(72) Erfinder: **Hoffmann, Michael, Dr.**
**Berliner Strasse 10**
**W-6238 Hofheim/Taunus (DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.09/3.3.3)

## Beschreibung

Die Erfindung betrifft Verfahren und Zwischenprodukte zur Herstellung von phosphorhaltigen L-Aminosäuren der Formel I oder deren Salze,

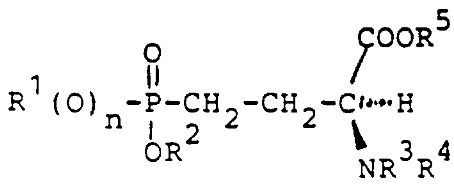

(I)

worin
$R^1$ und $R^2$ unabhängig voneinander Wasserstoff, $(C_1\text{-}C_6)$-Alkyl, das unsubstituiert ist oder durch Halogen oder Aryl ein- oder mehrfach substituiert ist, oder $(C_3\text{-}C_{10})$-Cycloalkyl,
$R^3$ und $R^4$ unabhängig voneinander Wasserstoff, $(C_1\text{-}C_6)$-Alkyl, $(C_2\text{-}C_6)$-Alkenyl, $(C_2\text{-}C_6)$-Alkinyl, wobei die 3 letztgenannten Reste geradkettig oder verzweigt sein können und Heteroatome in der Kette besitzen können, $(C_1\text{-}C_6)$-Alkylcarbonyl, Aryl-$(C_1\text{-}C_4)$-alkylcarbonyl, $(C_1\text{-}C_6)$-Alkoxycarbonyl, $(C_1\text{-}C_6)$-Alkylsulfonyl, Benzyl, Benzyloxycarbonyl, Aryloxycarbonyl oder Arylsulfonyl, wobei die vier letztgenannten Reste im Arylteil unsubstituiert oder substituiert sind,
$R^5$ Wasserstoff oder $(C_1\text{-}C_4)$-Alkyl, das geradkettig oder verzweigt ist und unsubstituiert oder durch Halogen, Aryl oder einen Heterocyclus substituiert ist, oder $(C_3\text{-}C_7)$-Cycloalkyl und
n die Zahl 0 oder 1 bedeuten.

Die Verbindungen der Formel I sind z. B. aus Bull. Chem. Soc. Japan 60, 1761 (1987) und DE-A-2 856 260 bekannt. Dort werden auch deren herbizide Eigenschaften beschrieben. Hervorzuheben unter den Verbindungen der Formel I sind die Verbindungen, worin $R^1(O)_n$ = $CH_3$ oder $C_2H_5$, $R^2O$ = OH sowie $R^3$, $R^4$ und $R^5$ jeweils Wasserstoff bedeuten, und deren Salze. Besondere Bedeutung besitzt die L-Form des Herbizids Glufosinate (4-[Hydroxy(methyl)phosphinoyl]-homoalanin und deren Salze, siehe D,L-Form in "The Pesticide Manual", 9th Ed. 1990, S. 458; siehe L-Form in DE-A-2856260).

Da gemaß DE-A-2856260 die herbizide Wirkung des L-Isomeren doppelt so stark wie die des Racemats ist, bietet der Einsatz des L-Isomeren eindeutige Vorteile, z. B. reduzierte Aufwandmenge, geringere Nebenwirkungen etc.

Phosphorhaltige L-Aminosäuren können mit hoher optischer Reinheit bisher durch aufwendige, enzymatische Racematspaltungsverfahren erhalten werden (DE-A-2939265 und DE-A-3048612).

Daneben gibt es Verfahren, die eine Transaminierung der zugrundeliegenden phosphorhaltigen $\alpha$-Ketocarbonsäuren (d. h. wenn in Formel I eine Carbonylgruppe anstelle von $CHNR^3R^4$ steht) mit Mikroorganismen bzw. deren Transaminasen beschreiben (EP-A-248357, EP-A-249188). Ein Nachteil dieser Verfahren liegt in der geringen Raum-Zeit-Ausbeute, der technisch problematischen Aufarbeitung der Rohlösung und in der schwierigen Reinigung der Endprodukte.

Weiterhin gibt es auch Verfahren für enantioselektive Synthesen von L-Aminosäurederivaten, welche jedoch verschiedene Nachteile besitzen. So ergibt z. B. die in EP-A-127429 beschriebene enantioselektive Alkylierung von chiralen Schiff-Basen nur optische Ausbeuten von maximal 78 %, während die in DE-A-3609818 beschriebene asymmetrische Hydrierung von schwer zugänglichen 2,3-Dehydroaminosäuren und Katalysatoren ausgeht.

Daneben sind Verfahren bekannt, die von heterocyclischen Vorstufen ausgehen (DE-A-3542645 und DE-A-3525267). Diese Verfahren haben jedoch den Nachteil, daß die betreffenden heterocyclischen Ausgangsverbindungen nur in mehrstufigen und aufwendigen Syntheseschritten herstellbar sind.

Weiterhin ist ein Verfahren bekannt, welches von L-Glutaminsäure ausgeht (DE-A-3817956). Ein Nachteil bei diesem Verfahren liegt in der geringen Ausbeute bei der Herstellung von Vinylglycinderivaten, welche die zentralen Zwischenverbindungen bei dieser Synthese darstellen (Tetrahedron Lett. 1984, 1425).

In der deutschen Patentanmeldung P 4103821.5 wurde bereits ein einfaches Verfahren zur Herstellung von L-Homoserinlactonen aus der preiswerten und gut zugänglichen L-Asparaginsäure vorgeschlagen; es wäre deshalb von Vorteil L-Homoserinlactone als Zwischenprodukte zur Herstellung der phosphorhaltigen L-Aminosäuren der Formel I einsetzen zu können.

Es wurden nun Verfahren zur Herstellung der Verbindungen der Formel I gefunden, welche die obengenannten Nachteile vermeiden und von gut zugänglichen optisch aktiven Ausgangsstoffen ausgehen.

Gegenstand der Erfindung ist ein Verfahren zur Herstellung einer phosphorhaltigen L-Aminosäuren der genannten Formel I oder deren Salzen, dadurch gekennzeichnet, daß man

a) ein optisch aktives S-Homoserinlacton der Formel II,

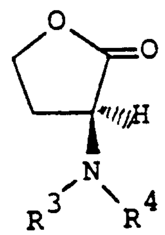

(II)

worin

$R^3$ und $R^4$ wie in Formel I definiert sind, in Gegenwart eines Alkohols der Formel $R^5OH$ mit Chlorwasserstoff zu einer Verbindung der Formel III,

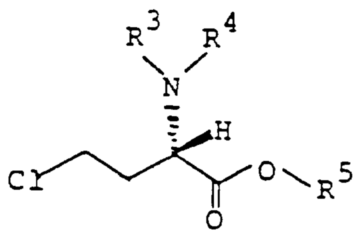

(III)

worin

$R^3$, $R^4$ und $R^5$ wie in Formel I definiert sind, ausgenommen $R^5$ = H, umsetzt und

b) die erhaltene Verbindung der Formel III mit einer Verbindung der Formel IV,

$R^1(O)_n$ - $P(OR^2)_2$ (IV)

worin

$R^1$, $R^2$ und n wie in Formel I definiert sind, umsetzt und für den Fall, daß die Verbindung I mit $R^5$ = H hergestellt wird, die zunächst erhaltene Verbindung I hydrolysiert.

In den genannten Formeln und im folgenden bedeutet Aryl, auch in den zusammengesetzten Resten wie Arylcarbonyl, vorzugsweise Phenyl; substituiertes Aryl oder Benzyl ist vorzugsweise Phenyl bzw. Benzyl, die durch ein oder mehrere Reste aus der Gruppe $(C_1-C_4)$-Alkyl, $(C_1-C_4)$-Alkoxy, Halogen, Nitro, $(C_1-C_4)$-Alkoxycarbonyl, Amino, $(C_1-C_4)$-Alkylamino und $(C_1-C_4)$-Dialkylamino, insbesondere aus der Gruppe Methyl, Ethyl, Methoxy, Ethoxy und Halogen, substituiert sind. Halogen bedeutet in den Formeln Fluor, Chlor, Brom oder Jod.

Alkyl, Alkenyl und Alkinyl, welche Heteroatome in der Kette enthalten können, sind beispielsweise solche Reste mit Sauerstoffatomen oder Schwefelatomen als Heteroatomen, vorzugsweise Polyoxalkylenreste mit 2 bis 5 Alkylenoxyeinheiten.

Heterocyclus ist beispielsweise ein gegebenenfalls substituierter, aromatischer ungesättigter oder gesättigter Ring mit 3 bis 7 Ringatomen, der 1 bis 4 Heteroatome aus der Gruppe O, S und N enthalten kann. Haloalkyl ist Alkyl, welches durch ein oder mehrere Halogenatome substituiert ist.

Gegenstand der Erfindung sind auch die Verfahren, die den einzelnen Verfahrensstufen a) und b) des obengenannten Gesamtverfahrens entsprechen.

Das erfindungsgemäße Gesamtverfahren stellt somit eine Lactonringöffnung von optisch aktiven L-Homoserinlactonen der Formel II, welche aus L-Asparaginsäure leicht zugänglich sind, mit gasförmigem Chlorwasserstoff und eine anschließende Umsetzung der so erhaltenen Chloride der Formel III mit Verbindungen der Formel IV zu den phosphorhaltigen L-Aminosäuren der Formel I dar.

Die Ausgangsstoffe der Formel II sind nach literaturbekannten Methoden aus Homoserinlactonen der Formel V

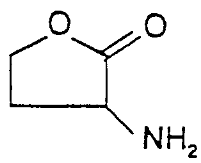

(V)

leicht zugänglich (Houben-Weyl, Band 15/1, 46-305).

Weiterhin sind Ausgangsstoffe der Formel II, worin ein Rest der Reste $R^3$ und $R^4$ Wasserstoff bedeutet, auf einfache Weise aus Asparaginsäurederivaten der Formel VI,

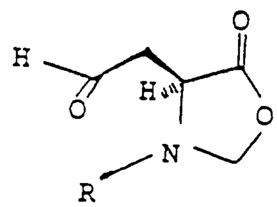

(VI)

worin R die für $R^3$ oder $R^4$ genannte Bedeutung hat, leicht zugänglich (siehe P4103821.5).

Bevorzugt sind erfindungsgemäße Verfahren, in denen $R^1$ und $R^2$ unabhängig voneinander ($C_1$-$C_4$)-Alkyl, ($C_1$-$C_4$)-Haloalkyl, Benzyl, 1- oder 2-Phenylethyl, Cyclopentyl oder Cyclohexyl, insbesondere Methyl oder Ethyl, $R^3$ und $R^4$ unabhängig voneinander ($C_1$-$C_4$)-Alkyl, ($C_1$-$C_4$)-Alkylcarbonyl, Phenacetyl, ($C_1$-$C_4$)-Alkoxycarbonyl, ($C_1$-$C_4$)-Alkylsulfonyl, Benzyl, Benzyloxycarbonyl, Phenoxycarbonyl, Benzolsulfonyl, wobei die 4 letztgenannten Reste im Phenylring unsubstituiert oder durch Reste aus der Gruppe ($C_1$-$C_3$)-Alkyl, ($C_1$-$C_2$)-Alkoxy und Halogen substituiert sind, $R^5$ H, ($C_1$-$C_4$)-Alkyl, ($C_1$-$C_4$)-Haloalkyl, Phenyl-($C_1$-$C_4$)-alkyl, Cyclopentyl oder Cyclohexyl und n 0 oder 1 bedeuten.

Das erfindungsgemäße Verfahren wird in Verfahrensstufe a) z. B. so durchgeführt, daß man die Lactone der Formel II in einem geeigneten aliphatischen, cycloaliphatischen, araliphatischen oder heterocyclisch substituierten aliphatischen Alkohol, wie z. B. Methanol, Ethanol, n-Propanol, i-Propanol und Cyclohexanol, bei Temperaturen von 0 °C bis Rückflußtemperatur, vorzugsweise 40 bis 70 °C, mit Chlorwasserstoffgas behandelt.

Die Herstellung einiger $\gamma$-Halogen-$\alpha$-amino-buttersäurederivate durch Ringöffnungsreaktionen von Homoserinlactonen mit gasförmigen Halogenwasserstoffen ist bekannt. Die bekannten Synthesen sind aber mit verschiedenen Nachteilen behaftet.

Bei den meisten Verfahren wird zur Lactonringöffnung Bromwasserstoff eingesetzt (Chemistry Lett., 1973, 5; Tetrahedron Lett., 1986, 27, 5935). Diese Methode erfordert recht stabile Schutzgruppen an der Aminogruppe. Viele Schutzgruppen halten diesen sauren Bedingungen nicht stand, was schließlich zu Ausbeuteverlusten und Produktgemischen führt (Tetrahedron 44 (1988), 637). Auch bei Ringöffnungsreaktionen mit Chlorwasserstoff werden bestimmte Reste am Stickstoff abgespalten (J. Chem. Soc. 1958, 1629). Da es sich hierbei zum Teil auch um Schutzgruppen handelt, die erst später unter bestimmten Bedingungen gezielt entfernt werden sollen, sind solche Abspaltungsreaktionen nicht wünschenswert.

Überraschenderweise gelingt es nach dem erfindungsgemäßen Verfahren, mit Hilfe des preiswerteren Chlorwasserstoffs anstelle von Bromwasserstoff die gewünschte Ringöffnung ohne Racemisierung und ohne Abspaltung von Schutzgruppen durchzuführen. Die praktisch racemisierungsfreie Ringöffnung der Verbindungen der Formel II zu den Verbindungen der Formel III ist umso überraschender, weil bei Verwendung von Jodwasserstoff teilweise Racemisierung stattfindet (siehe Tetrahedron Lett. 1984, 2231).

Die optisch aktiven Verbindungen der Formel III sind nicht nur als Zwischenprodukte für das erfindungsgemäße Verfahren einsetzbar, sondern stellen wertvolle Synthone zur Herstellung von optisch aktiven und biologisch wirksamen Verbindungen dar. So können sie zum Beispiel in die optisch aktiven Azetidin-2-carbonsäurederivate überführt werden (JP-49014457). Weiterhin können sie bei der Synthese von Nocardicin, einem $\beta$-Lactam-antibiotikum, Verwendung finden (J.Am.Chem.Soc. 112 (1990), 760-770). Man kann sie auch zur Herstellung von wichtigen Aminosäuren, wie z. B. L-Canalin (Liebigs Ann.Chem. 1986, 287-291 und dort zit. Lit.) oder schwefelhaltigen Aminosäuren (DE-A-2627069) einsetzen. Die erfindungsgemäß in Verfahrensstufe a) erhaltenen Verbindungen der Formel III können selektiv nach üblichen Methoden an der Estergruppe zur entsprechenden Carbonsäure hydrolysiert und weiter zu Salzen mit Basen umgesetzt

werden.
Außerdem können die Ester und Carbonsäuren der Formel III Säureadditionssalze bilden.
Die neuen Verbindungen der genannten Formel III und ihre Salze sind deshalb auch Gegenstand dieser Erfindung. Die Verbindung der Formel III, worin $R^3$, $R^4$, $R^5$ jeweils H bedeuten, ist aus DE-A-2627069 bereits bekannt.
Die Verbindung der Formel III, worin einer der Reste $R^3$ und $R^4$ = t-Butyloxycarbonyl und der andere der Reste $R^3$ und $R^4$ = H sowie $R^5$ = Benzyl bedeuten, ist aus Tetrahedron 44 (1988) 5479-86 bekannt.

Die erfindungsgemäß erhaltenen Chloride der Formel III werden ohne Zwischenisolierung oder vorzugsweise nach Aufarbeitung und gegebenenfalls Reinigung in Verfahrensstufe b) mit Phosphorverbindungen der Formel IV beispielsweise in Substanz oder in organischen Lösungsmitteln, wie aliphatischen oder aromatischen, gegebenenfalls halogenierten Kohlenwasserstoffen, z. B. Benzol oder Toluol, bei Temperaturen von vorzugsweise 50 °C - 150 °C, vorzugsweise in Substanz (ohne Lösungsmittel) bei 110 bis 140 °C zu den Verbindungen der Formel I umgesetzt. Auf diese Weise werden vorzugsweise Verbindungen der Formel I erhalten, worin $R^1$, $R^2$, $R^3$ und/oder $R^4$ und $R^5$ ungleich Wasserstoff sind.

Arbuzovreaktionen von Phosphorverbindungen der Formel IV mit Halogeniden, die sich strukturell von denen der Formel III unterscheiden, sind bekannt (Chem. Rev. 81 (1981) 415-430 und dort zit. Lit.). Weiterhin sind Arbuzovreaktionen mit racemischen 4-Brom-2-phthalimido-buttersäureethylester bekannt (Tetrahedron Lett., 1986, 5935).

Überraschend an der erfindungsgemäßen Verfahrensstufe b) ist aber beispielsweise, daß man die Arbuzovreaktion mit den Chloriden der Formel III durchführen kann, obwohl Alkylchloride weitaus weniger reaktiv sind als die entsprechenden Bromide (Chem. Rev. 81 (1981) 415-430 und dort zit. Lit.), und daß bei dieser Arbuzovreaktion trotz der relativ hohen Reaktionstemperaturen keine Racemisierungen stattfinden.

Im Anschluß an die Umsetzung der Verbindungen der Formeln II und III können die erhaltenen Verbindungen der Formel I zu anderen Verbindungen der Formel I oder deren Salzen derivatisiert werden, beispielsweise durch Hydrolyse aller oder eines Teils der hydrolytisch abspaltbaren Reste und gegebenenfalls Salzbildung mit Basen oder Säuren. Ein günstiges Hydrolyseverfahren ist beispielsweise die saure Hydrolyse von Verbindungen der Formel I, worin $R^1$, $R^2$ und $R^5$ sowie mindestens einer Rest der beiden Reste $R^3$ und $R^4$ ungleich Wasserstoff sind, zu Verbindungen der Formel I, worin $R^1$ die genannte Bedeutung hat und $R^2$ bis $R^5$ jeweils Wasserstoff sind. Zur Hydrolyse werden dabei die zunächst erhaltenen Verbindungen der Formel I vorzugsweise in wässeriger Salzsäure (bevorzugt 3N-12N HCl) gelöst und mehrere Stunden auf 90 °C bis 130 °C erhitzt. Die saure, wäßrige Lösung wird dann mit einem mit Wasser nicht oder kaum mischbaren organischen Lösungsmittel, wie z. B. Toluol, Xylol, Dichlormethan oder Methylisobutylketon, extrahiert, um die Folgeprodukte der Abspaltung zu entfernen. Die wässrige Lösung wird vollständig eingeengt und, wenn nötig, das Rohprodukt nach bekannten Methoden gereinigt. Falls erwünscht, können die so erhaltenen Hydrochloride der Verbindungen der allgemeinen Formel I auf üblichem Wege in die freien Aminosäuren überführt werden.

Zur Überführung der Verbindungen der Formel I in ihre Salze werden solche mit einem aciden Wasserstoffatom beispielsweise mit Basen, vorzugsweise Alkalimetall- oder Erdalkalimetallhydroxiden, -carbonaten oder -hydrogencarbonaten oder organischen Aminen, zu im Pflanzenschutz einsetzbaren Salzen umgesetzt.

Außerdem können aufgrund der enthaltenen Aminogruppe Säureadditionssalze, beispielsweise mit Mineralsäuren wie Schwefelsäure, Salzsäure, Bromwasserstoffsäure, Jodwasserstoffsäure, Fluorwasserstoffsäure und Phosphorsäure, oder mit organischen Säuren, wie Ameisensäure, Essigsäure, Milchsäure und Citronensäure, hergestellt werden.

Das erfindungsgemäße Verfahren liefert die L-Isomeren der Formel I in hohen optischen Ausbeuten von über 90 % ee, was einem Anteil von mehr als 95 % des L-Isomeren entspricht. Die Konfiguration der L-Formen (Fischer-Projektion) entspricht in der R,S-Nomenklatur (Cahn, Ingold und Prelog) der S-Konfiguration.

Das erfindungsgemäße Verfahren ermöglicht somit insbesondere die Herstellung optisch aktiver $\gamma$-Chlor-$\alpha$-aminobuttersäurederivate der Formel III aus einfach zugänglichen und optisch aktiven Homoserinlactonen der Formel II und die Herstellung optisch aktiver phosphorhaltiger L-Aminosäuren der Formel I aus den leicht zugänglichen Chlorverbindungen der Formel III.

Da die Homoserinlactone der Formel II aus leicht zugänglichen L-Asparaginsäurederivaten hergestellt werden können, bedeutet das erfindungsgemäße Verfahren eine günstige Herstellung von phosphorhaltigen L-Aminosäuren der Formel I unter Nutzung des Naturprodukts L-Asparaginsäure als Chiral-Pool-Substanz.

BEISPIEL 1

(S)-2-(Tosyl-amino)-4-chlor-buttersäureethylester

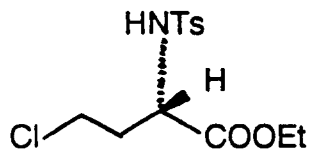

7,5 g (29,4 mmol) N-p-Toluolsulfonyl-L-homoserinlacton ( = S-Form), hergestellt nach P 4103821.5 werden in 60 ml Ethanol gelöst und auf 60 °C - 70 °C erwärmt. Danach leitet man 4 h lang Chlorwasserstoffgas ein und läßt die Reaktionslösung anschließend 12 h bei Raumtemperatur stehen. Danach engt man bis zur Trockne ein und reinigt das Rohprodukt säulenchromatographisch an Kieselgel (Laufmittel: Heptan/Essigsäureethylester = 3/7); man erhält 8 g (89 % d. Th.) Produkt als farbloses Öl, das nach längerem Stehen zu kristallisieren beginnt. Charakteristische Daten des Produkts sind:
Spezifische Drehung: $[\alpha]_D^{25}$ = 10,4 (c = 1, $CH_3OH$);
$^1$H-NMR (100 MHz): $\delta$ [ppm] = 7,75 und 7,26 (2m, 4H, Aromaten-H in -$C_6H_4$-$CH_3$); 5,35 (bd, 1H, NH); 4,10 (m, 1H, CH-N); 4,00 (q, 2H, $COOCH_2$-$CH_3$); 3,65 (t, 2H, -$CH_2Cl$); 2,40 (s, 3H, -$C_6H_4$-$CH_3$); 2,15 (m, 2H, -$CH_2$-$CH_2Cl$); 1,10 (t, 3H, -$COOCH_2CH_3$).

BEISPIEL 2

(S)-2-(Methoxycarbonyl-amino)-4-chlor-buttersäureethylester

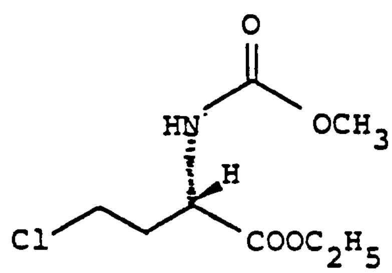

4 g (25 mmol) N-Methorycarbonyl-L-homoserinlacton ( = S-Form), hergestellt nach P 4103821.5 werden in 50 ml Ethanol gelöst und auf 60 °C erwärmt. Danach leitet man 6 h lang Chlorwasserstoffgas ein. Danach engt man bis zur Trockne ein und reinigt das Rohprodukt säulenchromatographisch an Kieselgel (Laufmittel: Heptan/Essigsäureethylester = 1/1); man erhält 4,0 g (72 % d. Th.) Produkt als farbloses Öl mit den folgenden charakteristischen physikalischen Daten:
Drehwert: $[\alpha]_D^{25}$ = 5,4 (c = 1, $CH_2Cl_2$).
$^1$H-NMR (100MHz): $\delta$ [ppm] = 5,40 (bd, 1H, NH); 4,49 (m, 1H, CH-N); 4,22 (q, 2H, -$COOCH_2CH_3$); 3,70 (s, 3H, $COOCH_3$); 3,60 (t, 2H, -$CH_2$-$CH_2Cl$); 2,25 (m, 2H -$CH_2CH_2Cl$); 1,30 (t, 3H, -$COOCH_2CH_3$).

In analoger Weise zu Beispielen 1 und 2 werden die in der Tabelle 1 aufgeführten Verbindungen erhalten.

Tabelle 1: Verbindungen der Formel III

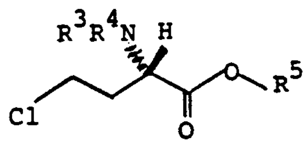

| Bsp. Nr.: | $R^3$ | $R^4$ | $R^5$ | Phys. Daten |
|---|---|---|---|---|
| 3 | H | $CH_3$ | $CH_3$ | |
| 4 | H | $C_2H_5$ | $CH_3$ | |
| 5 | H | $n\text{-}C_3H_7$ | $CH_3$ | |
| 6 | H | $i\text{-}C_3H_7$ | $CH_3$ | |
| 7 | H | $n\text{-}C_4H_9$ | $CH_3$ | |
| 8 | H | $CH_2\text{-}CH{=}CH_2$ | $CH_3$ | |
| 9 | H | $CH_2C{\equiv}CH$ | $CH_3$ | |
| 10 | H | $-CO\text{-}CH_3$ | $CH_3$ | |
| 11 | H | $-CO\text{-}C_2H_5$ | $CH_3$ | |
| 12 | H | $-CO\text{-}n\text{-}C_3H_7$ | $CH_3$ | |
| 13 | H | $-CO\text{-}OCH_3$ | $CH_3$ | |
| 14 | H | $-CO\text{-}OC_2H_5$ | $CH_3$ | |
| 15 | H | $-SO_2\text{-}CH_3$ | $CH_3$ | |
| 16 | H | $-SO_2\text{-}C_2H_5$ | $CH_3$ | |
| 17 | H | $-CH_2\text{-}C_6H_5$ | $CH_3$ | |
| 18 | H | $-CO\text{-}OCH_2\text{-}C_6H_5$ | $CH_3$ | |
| 19 | H | $-CO\text{-}OC_6H_5$ | $CH_3$ | |
| 20 | H | $-CO\text{-}O\text{-}p\text{-}C_6H_4CH_3$ | $CH_3$ | |
| 21 | H | $-SO_2\text{-}C_6H_5$ | $CH_3$ | |
| 22 | H | $-SO_2\text{-}p\text{-}C_6H_4CH_3$ | $CH_3$ | |
| 23 | H | $-CH_2CH_2\text{-}OCH_3$ | $CH_3$ | |
| 24 | H | $-CH(OCH_3)\text{-}CH_3$ | $CH_3$ | |
| 25 | H | $n\text{-}C_3H_7$ | $C_2H_5$ | |
| 26 | H | $i\text{-}C_3H_7$ | $C_2H_5$ | |

| Bsp. Nr.: | $R^3$ | $R^4$ | $R^5$ | Phys. Daten |
|---|---|---|---|---|
| 27 | H | n-$C_4H_9$ | $C_2H_5$ | |
| 28 | H | $CH_2$-$CH=CH_2$ | $C_2H_5$ | |
| 29 | H | $CH_2C\equiv CH$ | $C_2H_5$ | |
| 30 | H | -CO-$CH_3$ | $C_2H_5$ | |
| 31 | H | -CO-$C_2H_5$ | $C_2H_5$ | |
| 32 | H | -CO-H-$C_3H_7$ | $C_2H_5$ | |
| 33 | H | -CO-$OCH_3$ | $C_2H_5$ | |
| 34 | H | -CO-$OC_2H_5$ | $C_2H_5$ | |
| 35 | H | -$SO_2$-$CH_3$ | $C_2H_5$ | |
| 36 | H | -$SO_2$-$C_2H_5$ | $C_2H_5$ | |
| 37 | H | -$CH_2$-$C_6H_5$ | $C_2H_5$ | |
| 38 | H | -CO-$OCH_2C_6H_5$ | $C_2H_5$ | |
| 39 | H | -CO-$OC_6H_5$ | $C_2H_5$ | |
| 40 | H | -CO-O-p-$C_6H_4CH_3$ | $C_2H_5$ | |
| 41 | H | -$SO_2$-$C_6H_5$ | $C_2H_5$ | |
| 42 | H | -$SO_2$-p-$C_6H_4CH_3$ | $C_2H_5$ | |
| 43 | H | -$CH_2CH_2$-$OCH_3$ | $C_2H_5$ | |
| 44 | H | -$CH(OCH_3)$-$CH_3$ | $C_2H_5$ | |
| 55 | H | $CH_3$ | $C_2H_5$ | |
| 56 | H | $CH_3$ | $C_2H_5$ | |
| 57 | $CH_3$ | $CH_3$ | $CH_3$ | |
| 58 | $C_2H_5$ | $C_2H_5$ | $CH_3$ | |
| 59 | $CH_3$ | $CH_3$ | $C_2H_5$ | |
| 60 | $CH_3$ | -CO-$CH_3$ | $CH_3$ | |
| 61 | $CH_3$ | -CO-$C_2H_5$ | $CH_3$ | |
| 62 | H | -CO-i-$C_3H_7$ | $CH_3$ | |
| 63 | H | -CO-$OCH_3$ | n-$C_3H_7$ | |
| 64 | H | -CO-$OC_2H_5$ | n-$C_4H_9$ | |
| 65 | H | -$SO_2$-$CH_3$ | cyclo-$C_6H_{11}$ | |
| 66 | H | -$SO_2$-$C_2H_5$ | $CH_2CH_2Cl$ | |

| Bsp. Nr.: | $R^3$ | $R^4$ | $R^5$ | Phys. Daten |
|---|---|---|---|---|
| 67 | H | $-CH_2-C_6H_5$ | $CH_3$ | |
| 68 | H | $-CO-OCH_2-C_6H_5$ | $-CH_2-$ 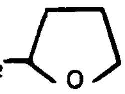 | |
| 69 | H | $-CO-OC_6H_5$ | $C_3H_7$ | |
| 70 | H | $-CO-O-p-C_6H_4CH_3$ | $C_3H_7$ | |
| 71 | H | $-SO_2-C_6H_5$ | $C_3H_7$ | |
| 72 | H | $-SO_2-p-C_6H_4CH_3$ | $C_3H_7$ | |
| 73 | H | $-CH_2CH_2-OCH_3$ | $C_3H_7$ | |
| 74 | H | $-CH(OCH_3)-CH_3$ | $C_3H_7$ | |

Beispiel 75:

(S)-2-(p-Toluolsulfonylamino)-4-[ethoxy(methyl)phosphinyl]-butansäureethylester

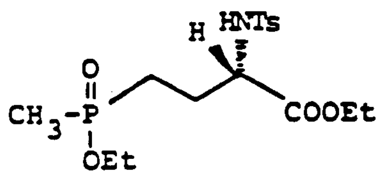

5g (16,35 mmol) (S)-2-(p-Toluolsulfonyl-amino)-4-chlor-butansäureethylester und 9 g (66,13 mmol) Methanphosphonigsäurediethylester werden 15 h bei 140 °C gerührt. Danach entfernt man den überschüssigen Methanphosphonigsäurediethylester am Hochvakuum. Das so erhaltene Rohprodukt zeigt im $^1$H-NMR keine weiteren organischen Verunreinigungen mehr und wird säulenchromatographisch an Kieselgel (Laufmittel: Methylenchlorid/Methanol = 95/5) weiter gereinigt; man erhält 4,1 g (65 %) Produkt als farbloses Öl mit folgenden physikalischen Daten:

$[\alpha]_D^{25}$ = + 8,7 (c = 1, MeOH);

$^1$H-NMR (100 MHz): $\delta$ [ppm] = 7,73 und 7,29 (2 bd, 4H, $-C_6H_4-CH_3$); 5,68 (bd, 1H, NH); 4,00 (m, 5H, CH-N, $COOCH_2CH_3$, $-POCH_2CH_2$); 2,40 (s, 3H, $-C_6H_4-CH_3$); 1,90 (m, 4H, $-CH_2-CH_2-$); 1,45 (d, 3H, $CH_3$-P); 1,30 und 1,13 (2t, 6H, $POCH_2CH_3$, $COOCH_2CH_3$).

Beispiel 76:

(S)-2-(Methoxycarbonylamino)-4-[ethoxy(methyl)phosphinyl]-butansäureethylester

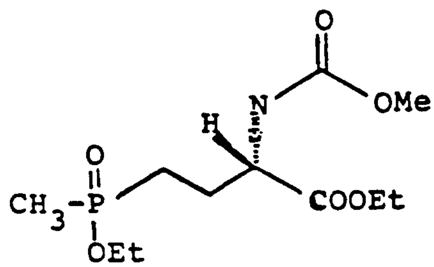

2,6 g (11,6 mmol) (S)-2-(Methoxycarbonyl-amino)-4-chlor-butansäureethylester und 6,3 g (46,4 mmol) Methanphosphonigsäurediethylester werden 15 h bei 140 °C gerührt. Danach entfernt man den überschüssigen Methanphosphonigsäurediethylester bei 90 °C am Hochvakuum. Das so erhaltene Rohprodukt fällt als farbloses Öl an und zeigt im $^1$H-NMR keine weiteren Verunreinigungen mehr; Ausbeute: 2,8 g (82 %), $^1$H-NMR (100 MHz) : δ [ppm] = 5,75 (bd, 1H, NH), 4,40 (m, 1H,CH-N); 4,19 und 4,00 (2q, 4H, $-COOCH_2CH_3$, $-POCH_2CH_3$); 3,67 (s, 3H, $COOCH_3$); 1,90 (m, 4H, - $CH_2CH_2$-); 1,44 (d, 3H, $CH_3P$); 1,29 und 1,26 (2t, 6H, $POCH_2CH_3$, $COOCH_2CH_3$).

Beispiel 77:

L-Phosphinothricin-Hydrochlorid

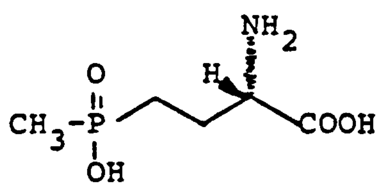 x HCl

2,8 g (9,5 mmol) (S)-2-Methoxycarbonylamino-4-ethoxy(methyl)-phosphinylbutansäureethylester werden in 50 ml 6N HCl 12 h am Rückfluß gekocht. Danach wird die wässrige Lösung mit Dichlormethan ertrahiert, mit wenig Aktivkohle versetzt, kurz aufgekocht und nach Abfiltrieren der Aktivkohle bis zur Trockne eingeengt. Der Rückstand wird mehrmals in Toluol/Aceton aufgenommen und eingeengt. Man erhält 1,5 g (73 % d. Th.) Produkt als farblosen Feststoff; $^1$H-NMR (100 MHz, $D_2O$) : δ [ppm] = 4,16 (t, 1H, CH-N), 2,10 (m, 4H, $-CH_2CH_2-$); 1,55 (d, 3H, $CH_3$-P).

Der Enantiomerenüberschuß, der mit Hilfe einer HPLC Methode [J. Chromatogr. 368, 413 (1986)] bestimmt wurde, beträgt 94,2 % ee (ee = enantiomeric excess).

**Patentansprüche**

**1.** Verfahren zur Herstellung von Verbindungen der Formel I oder deren Salze,

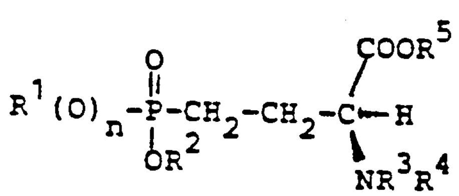 (I)

worin
$R^1$ und $R^2$ unabhängig voneinander Wasserstoff, $(C_1\text{-}C_6)$-Alkyl, das unsubstituiert ist oder durch Halogen oder Aryl ein- oder mehrfach substituiert ist, oder $(C_3\text{-}C_{10})$-Cycloalkyl,
$R^3$ und $R^4$ unabhängig voneinander Wasserstoff, $(C_1\text{-}C_6)$-Alkyl, $(C_2\text{-}C_6)$-Alkenyl, $(C_2\text{-}C_6)$-Alkinyl, wobei die drei letztgenannten Reste geradkettig oder verzweigt sein können und Heteroatome in der Kette besitzen können, $(C_1\text{-}C_6)$-Alkylcarbonyl, Aryl-$(C_1\text{-}C_4)$-alkylcarbonyl, $(C_1\text{-}C_6)$-Alkoxycarbonyl, $(C_1\text{-}C_6)$-Alkylsulfonyl, Benzyl, Benzyloxycarbonyl, Aryloxycarbonyl oder Arylsulfonyl, wobei die vier letztgenannten Reste im Arylteil unsubstituiert oder substituiert sind,
$R^5$ Wasserstoff oder $(C_1\text{-}C_4)$-Alkyl, das geradkettig oder verzweigt ist und unsubstituiert oder durch Halogen, Aryl oder einen Heterocyclus substituiert ist, oder $(C_3\text{-}C_7)$-Cycloalkyl und
n die Zahl 0 oder 1 bedeuten
dadurch gekennzeichnet, daß man

a) ein optisch aktives S-Homoserinlacton der Formel II,

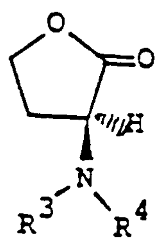

(II)

worin
$R^3$ und $R^4$ wie in Formel I definiert sind, in Gegenwart eines Alkohols der Formel $R^5$-OH mit Chlorwasserstoff zu einer Verbindung der Formel III,

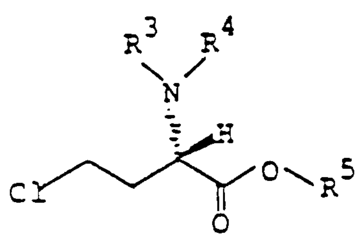

(III)

worin
$R^3$, $R^4$ und $R^5$ wie in Formel I definiert sind, ausgenommen $R^5$ = H, umsetzt und

b) die erhaltene Verbindung der Formel III mit einer Verbindung der Formel IV,

$R^1(O)_n$ - $P(OR^2)_2$ (IV)

worin
$R^1$, $R^2$ und n wie in Formel I definiert sind, umsetzt und für den Fall, daß die Verbindung I mit $R^5$ = H hergestellt wird, die zunächst erhaltene Verbindung I hydrolysiert.

**2.** Verfahren nach Anspruch 1, dadurch gekennzeichnet, daß
$R^1$ und $R^2$ unabhängig voneinander $(C_1\text{-}C_4)$-Alkyl, $(C_1\text{-}C_4)$-Haloalkyl, Benzyl, 1- oder 2-Phenylethyl, Cyclopentyl oder Cyclohexyl,
$R^3$ und $R^4$ unabhängig voneinander $(C_1\text{-}C_4)$-Alkyl, $(C_1\text{-}C_4)$-Alkylcarbonyl, Phenacetyl, $(C_1\text{-}C_4)$-Alkoxycarbonyl, $(C_1\text{-}C_4)$-Alkylsulfonyl, Benzyl, Benzyloxycarbonyl, Phenoxycarbonyl, Benzolsulfonyl, wobei die 4 letztgenannten Reste im Phenylring unsubstituiert oder durch Reste aus der Gruppe $(C_1C_2)$-Alkoxy und Halogen substituiert sind,
$R^5$ H, $(C_1\text{-}C_4)$-Alkyl, $(C_1\text{-}C_4)$-Haloalkyl, Phenyl-$(C_1\text{-}C_4)$-alkyl, Cyclopentyl oder Cyclohexyl und
n 0 oder 1 bedeuten.

**3.** Verfahren nach Anspruch 1 oder 2, dadurch gekennzeichnet, daß man in Verfahrensstufe a) die Umsetzung bei 0 °C bis zur Rückflußtemperatur durchführt.

**4.** Verfahren nach Anspruch 3, dadurch gekennzeichnet, daß die Temperatur 40 bis 70 °C beträgt.

**5.** Verfahren nach einem oder mehreren der Ansprüche 1 bis 4, dadurch gekennzeichnet, daß der Alkohol $R^5$-OH in Verfahrensstufe a) Methanol, Ethanol, n-Propanol, i-Propanol oder Cyclohexanol ist.

**6.** Verfahren nach einem oder mehreren der Ansprüche 1 bis 5, dadurch gekennzeichnet, daß die Umsetzung in Verfahrensstufe b) in Substanz bei 50 bis 150 °C durchgeführt wird.

**7.** Verbindungen der Formel III und ihre Salze,

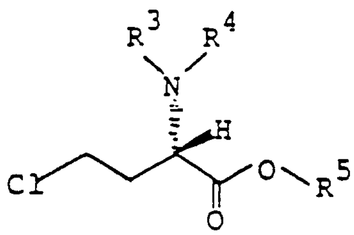

(III)

worin
$R^3$, $R^4$ und $R^5$ die in Formel I nach Anspruch 1 oder 2 definierte Bedeutung haben, ausgenommen die Verbindungen der Formel III, worin
$R^3$ = H, $R^4$ = H und $R^5$ = H bedeuten oder einer der Reste $R^3$ und $R^4$ = t-Butyloxycarbonyl und der andere der Reste $R^3$ und $R^4$ = H sowie $R^5$ = Benzyl bedeuten.

**8.** Verfahren zur Herstellung von Verbindungen der Formel III und deren Salze,

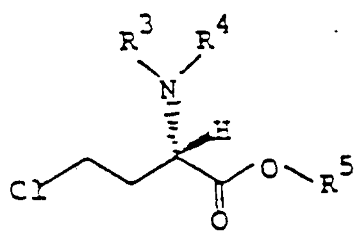

(III)

worin $R^3$, $R^4$ und $R^5$ die in Formel I nach Anspruch 1 oder 2 definierte Bedeutung haben, dadurch gekennzeichnet, daß man eine Verbindung der Formel II,

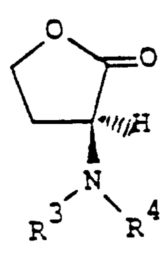

(II)

worin
$R^3$ und $R^4$ wie in Formel III definiert sind, in Gegenwart eines Alkohols der Formel $R^5$-OH, worin $R^5$ wie in Formel III definiert ist, ausgenommen $R^5$ = H, mit Chlorwasserstoff umsetzt und für den Fall, daß die

Verbindung III mit $R^5$ = H hergestellt wird, die zunächst erhaltene Verbindung III hydrolysiert.

**9.** Verfahren nach Anspruch 8, dadurch gekennzeichnet, daß man die Umsetzung bei 0 °C bis zur Rückflußtemperatur durchführt.

**10.** Verfahren nach Anspruch 9, dadurch gekennzeichnet, daß die Temperatur 40 bis 70 °C beträgt.

**11.** Verfahren nach einem oder mehreren der Ansprüche 8 bis 10, dadurch gekennzeichnet, daß der Alkohol $R^5$-OH Methanol, Ethanol, n-Propanol, i-Propanol oder Cyclohexanol ist.

**12.** Verfahren zur Herstellung von Verbindungen der Formel I oder deren Salze, wie sie in Anspruch 1 oder 2 definiert sind, dadurch gekennzeichnet, daß man eine Verbindung der Formel III,

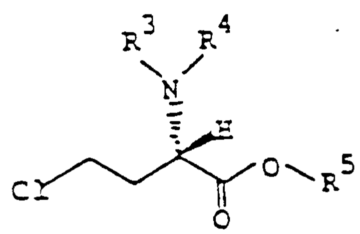

(III)

worin
$R^3$, $R^4$ und $R^5$ wie in Formel I, außer $R^5$ = H, definiert sind, mit einer Verbindung der Formel IV,

$R^1(O)_n$-$P(OR^2)_2$ (IV)

worin $R^1$ und $R^2$ wie in Formel I definiert sind, umsetzt und für den Fall, daß die Verbindung I mit $R^5$ = H hergestellt wird, die zunächst erhaltene Verbindung I hydrolysiert.

**13.** Verfahren nach Anspruch 12, dadurch gekennzeichnet, daß die Umsetzung bei 50 bis 150 °C durchgeführt wird.

**14.** Verfahren nach Anspruch 12 oder 13, dadurch gekennzeichnet, daß die Umsetzung in Substanz durchgeführt wird.

**15.** Verfahren nach Anspruch 14, dadurch gekennzeichnet, daß die Umsetzung bei 110 bis 140 °C durchgeführt wird.

**16.** Verfahren nach einem oder mehreren der Ansprüche 12 bis 15, dadurch gekennzeichnet, daß die erhaltene Verbindung der Formel I mit 3N bis 12N wäßriger Salzsäure bei 90 bis 130 °C zur Verbindung der Formel I, worin $R^5$ = H ist, hydrolysiert wird.

**17.** Verwendung von Verbindungen der Formel III, wie sie in Anspruch 8 definiert sind zur Herstellung von optisch aktiven Verbindungen der Formel I oder deren Salzen, wie sie in Formel I nach Anspruch 1 oder 2 definiert sind.

## Claims

1. A process for the preparation of a compound of the formula I or salt thereof,

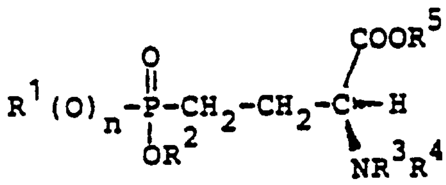

(I)

in which
$R^1$ and $R^2$ independently of each other are hydrogen, $(C_1\text{-}C_6)$-alkyl, which is unsubstituted or is mono- or polysubstituted by halogen or aryl, or $(C_3\text{-}C_{10})$-cycloalkyl,
$R^3$ and $R^4$ independently of each other are hydrogen, $(C_1\text{-}C_6)$-alkyl, $(C_2\text{-}C_6)$-alkenyl, $(C_2\text{-}C_6)$-alkynyl, where the last three radicals mentioned can be straight-chain or branched and can have hetero atoms in the chain, $(C_1\text{-}C_6)$-alkylcarbonyl, aryl-$(C_1\text{-}C_4)$-alkylcarbonyl, $(C_1\text{-}C_6)$-alkoxycarbonyl, $(C_1\text{-}C_6)$-alkylsulfonyl, benzyl, benzyloxycarbonyl, aryloxycarbonyl or arylsulfonyl, where the last four radicals mentioned are unsubstituted or substituted in the aryl moiety,
$R^5$ is hydrogen or $(C_1\text{-}C_4)$-alkyl, which is straight-chain or branched and is unsubstituted or substituted by halogen, aryl or a heterocycle, or $(C_3\text{-}C_7)$-cycloalkyl and
n is the number 0 or 1,
which comprises

a) reacting an optically active S-homoserine lactone of the formula II,

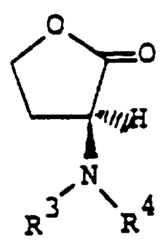

(II)

in which
$R^3$ and $R^4$ are defined as in formula I, with hydrogen chloride in the presence of an alcohol of the formula $R^5$-OH to give a compound of the formula III,

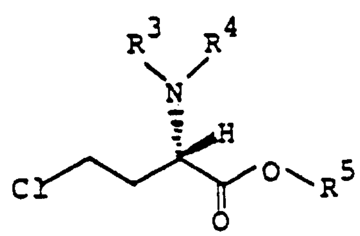

(III)

in which
$R^3$, $R^4$ and $R^5$ are defined as in formula I, with the exception of $R^5$ = H, and

b) reacting the resulting compound of the formula III with a compound of the formula IV,

$R^1(O)_n$ - $P(OR^2)_2$ (IV)

in which

$R^1$, $R^2$ and n are defined as in formula I and, if the compound I, in which $R^5$ = H, is being prepared, hydrolyzing the compound I initially obtained.

**2.** The process as claimed in claim 1, wherein
$R^1$ and $R^2$ independently of each other are $(C_1-C_4)$-alkyl, $(C_1-C_4)$-haloalkyl, benzyl, 1- or 2-phenylethyl, cyclopentyl or cyclohexyl,
$R^3$ and $R^4$ are independently of each other $(C_1-C_4)$-alkyl, $(C_1-C_4)$-alkylcarbonyl, phenacetyl, $(C_1-C_4)$-alkoxycarbonyl, $(C_1-C_4)$-alkylsulfonyl, benzyl, benzyloxycarbonyl, phenoxycarbonyl, benzenesulfonyl, where the last 4 radicals mentioned are unsubstituted in the phenyl ring or substituted in the phenyl ring by radicals selected from the group comprising $(C_1-C_2)$-alkoxy and halogen,
$R^5$ is H, $(C_1-C_4)$-alkyl, $(C_1-C_4)$-haloalkyl, phenyl-$(C_1-C_4)$-alkyl, cyclopentyl or cyclohexyl and
n is 0 or 1.

**3.** The process as claimed in claim 1 or 2, wherein in process step a) the reaction is carried out at 0 °C up to the reflux temperature.

**4.** The process as claimed in claim 3, wherein the temperature is 40 to 70 °C.

**5.** The process as claimed in one or more of claims 1 to 4, wherein the alcohol $R^5$-OH in process step a) is methanol, ethanol, n-propanol, i-propanol or cyclohexanol.

**6.** The process as claimed in one or more of claims 1 to 5, wherein the reaction in process step b) is carried out in the absence of solvent at 50 to 150 °C.

**7.** A compound of the formula III and salt thereof,

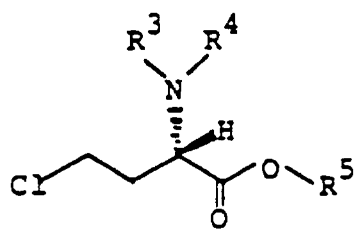

(III)

in which
$R^3$, $R^4$ and $R^5$ have the meaning defined in formula I as claimed in claim 1 or 2, with the exception of the compounds of the formula III in which $R^3$ = H, $R^4$ = H and $R^5$ = H or one of the radicals $R^3$ and $R^4$ = t-butyloxycarbonyl and the other of the radicals $R^3$ and $R^4$ = H and also $R^5$ = benzyl.

**8.** A process for the preparation of a compound of the formula III and salt thereof, which comprises reacting a compound of the formula II,

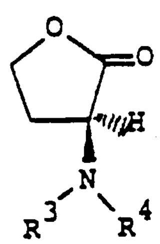

(II)

in which $R^3$, $R^4$ and $R^5$ have the meaning defined in formula I as claimed in claim 1 or 2
in which
$R^3$ and $R^4$ are defined as in formula III, with hydrogen chloride in the presence of an alcohol of the formula $R^5$-OH, in which $R^5$ is defined as in formula III, with the exception of $R^5$ = H, and, if the

compound III in which $R^5$ = H, is being prepared, hydrolyzing the compound III initially obtained.

**9.** The process as claimed in claim 8, wherein the reaction is carried out at 0 °C up to the reflux temperature.

**10.** The process as claimed in claim 9, wherein the temperature is 40 to 70 °C.

**11.** The process as claimed in one or more of claims 8 to 10, wherein the alcohol $R^5$-OH is methanol, ethanol, n-propanol, i-propanol or cyclohexanol.

**12.** A process for the preparation of a compound of the formula I or salt thereof, as defined in claim 1 or 2, which comprises reacting a compound of the formula III,

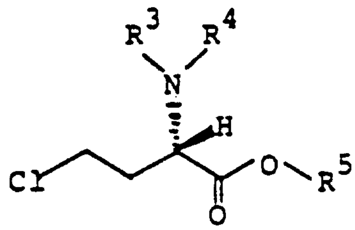

(III)

in which
$R^3$, $R^4$ and $R^5$ are defined as in formula I, except for $R^5$ = H, with a compound of the formula IV,

$R^1(O)_n$-$P(OR^2)_2$ (IV)

in which $R^1$ and $R^2$ are defined as in formula I, and, if the compound I in which $R^5$ = H, is being prepared, hydrolyzing the compound I initially obtained.

**13.** The process as claimed in claim 12, wherein the reaction is carried out at 50 to 150 °C.

**14.** The process as claimed in claim 12 or 13, wherein the reaction is carried out in the absence of solvent.

**15.** The process as claimed in claim 14, wherein the reaction is carried out at 110 to 140 °C.

**16.** The process as claimed in one or more of claims 12 to 15, wherein the compound obtained of the formula I is hydrolyzed with 3N to 12N aqueous hydrochloric acid at 90 to 130 °C to give the compound of the formula I, in which $R^5$ = H.

**17.** Use of a compound of the formula III as defined in claim 8 for the preparation of an optically active compound of the formula I or salt thereof, as defined in formula I fined in formula 1 in claim 1 or 2.

## Revendications

**1.** Procédé pour la préparation de composés de formule I ou de leurs sels :

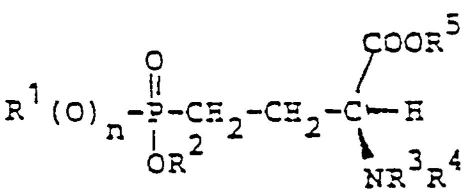

(I)

où

$R^1$ et $R^2$, indépendamment l'un de l'autre, représentent l'hydrogène, alkyle en $C_1$-$C_6$, qui est non substitué ou substitué par une ou plusieurs fois par halogène ou aryle, ou représentent cycloalkyle en $C_3$-$C_{10}$,
$R^3$ et $R^4$, indépendamment l'un de l'autre, représentent l'hydrogène, alkyle en $C_1$-$C_6$, alcényle en $C_2$-$C_6$, alcynyle en $C_2$-$C_6$, les trois derniers radicaux cités pouvant être linéaires ou ramifiés et contenir des hétéroatomes dans la chaîne, (alkyl en $C_1$-$C_6$)-carbonyle, aryl-(alkyl en $C_1$-$C_4$)-carbonyle, (alcoxy en $C_1$-$C_6$)-carbonyle, (alkyl en $C_1$-$C_6$)-sulfonyle, benzyle, benzyloxycarbonyle, aryloxycarbonyle ou arylsulfonyle, les quatre derniers dérivés cités pouvant être non substitués ou substitués dans la partie aryle,
$R^5$ représente l'hydrogène ou alkyle en $C_1$-$C_4$ qui est linéaire ou ramifié et non substitué ou substitué par halogène, aryle ou un hétérocycle, ou représente cycloalkyle en $C_3$-$C_7$ et
n vaut 0 ou 1,
caractérisé en ce qu'on fait réagir

a) une S-homosérinolactone optiquement active de formule II :

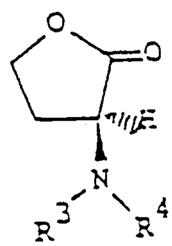

(II)

où
$R^3$ et $R^4$ sont définis comme dans la formule I, en présence d'un alcool de formule $R^5OH$, avec du chlorure d'hydrogène pour obtenir un composé de formule III

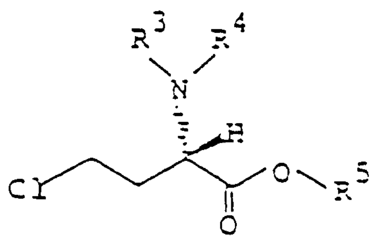

(III)

où
$R^3$, $R^4$ et $R^5$ sont définis comme dans la formule I sauf lorsque $R^5$ est égal à H, et

b) on fait réagir le composé obtenu de formule III avec un composé de formule IV :

$R^1(O)_n$ - $P(OR^2)_2$ (IV)

où
$R^1$, $R^2$ et n sont définis comme dans la formule I et dans le cas où on prépare le composé I avec $R^5$ représentant H, on hydrolyse le composé I obtenu initalement.

**2.** Procédé selon la revendication 1, caractérisé en ce que
$R^1$ et $R^2$, indépendamment l'un de l'autre, représentent alkyle en $C_1$-$C_4$, halogénoalkyle en $C_1$-$C_4$, benzyle, 1- ou 2-phényléthyle, cyclopentyle ou cyclohexyle, plus particulièrement méthyle et éthyle,
$R^3$ et $R^4$, indépendamment l'un de l'autre, représentent alkyle en $C_1$-$C_4$, (alkyl en $C_1$-$C_4$)-carbonyle, phénacétyle, (alcoxy en $C_1$-$C_4$)-carbonyle, (alkyl en $C_1$-$C_4$)-sulfonyle, benzyle, benzyloxycarbonyle, phénoxycarbonyle, benzènesulfonyle, les quatre derniers radicaux cités sont dans le cycle phényle non substitués ou substitués par des radicaux pris dans le groupe comportant alkyle en $C_1$-$C_3$, alcoxy en $C_1$-$C_2$ et halogène,

$R^5$ représente H, alkyle en $C_1$-$C_4$, halogénoalkyle en $C_1$-$C_4$, phényl-(alkyle en $C_1$-$C_4$), cyclopentyle ou cyclohexyle et
n vaut 0 ou 1.

**3.** Procédé selon la revendication 1 ou 2, caractérisé en ce qu'on effectue la réaction de l'étape de procédé a) à une température de 0 ° C jusqu'à la température de reflux.

**4.** Procédé selon la revendication 3, caractérisé en ce que la température est de 40 à 70 ° C.

**5.** Procédé selon une ou plusieurs des revendications 1 à 4, caractérisé en ce que l'alcool $R^5$-OH dans l'étape a) est le méthanol, l'éthanol, le n-propanol, l'i-propanol ou le cyclohexanol.

**6.** Procédé selon une ou plusieurs des revendications 1 à 5, caractérisé en ce qu'on effectue la réaction dans l'étape b) en substance à une température de 50 à 150 ° C.

**7.** Composé de formule III et leurs sels :

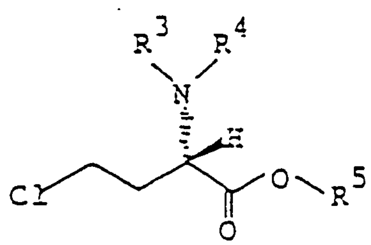

(III)

où
$R^3$, $R^4$ et $R^5$ dans la formule I ont la signification définie selon la revendication 1 ou 2, à l'exception des composés de formule III, où
$R^3$ correspond à H, $R^4$ correspond à H et $R^5$ correspond à H, ou
l'un des radicaux $R^3$ et $R^4$ est égal à t-butyloxycarbonyle et l'autre des radicaux $R^3$ et $R^4$ est égal à H ainsi que $R^5$ à benzyle.

**8.** Procédé pour la préparation de composés de formule III et leurs sels :

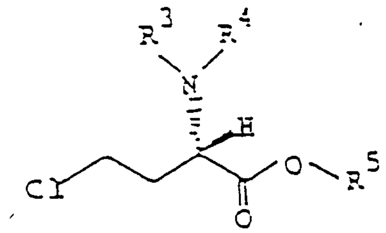

(III)

où
$R^3$, $R^4$ et $R^5$ dans la formule I ont la signification définie selon la revendication 1 ou 2, caractérisé en ce qu'on fait réagir un composé de formule II :

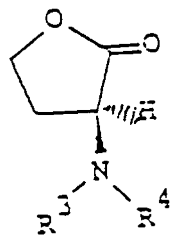

(II)

où

$R^3$ et $R^4$ sont définis comme dans la formule III, en présence d'un alcool de formule $R^5$-OH, où $R^5$ est défini comme dans la formule III, sauf lorsque $R^5$ est égal à H, avec du chlorure d'hydrogène et dans le cas où on prépare un composé III avec R égal à H, on hydrolyse le composé III obtenu initialement.

**9.** Procédé selon la revendication 8, caractérisé en ce qu'on met en oeuvre la réaction à des températures de 0 °C jusqu'à la température de reflux.

**10.** Procédé selon la revendication 9, caractérisé en ce que la température est de 40 à 70 °C.

**11.** Procédé selon une ou plusieurs des revendications 8 à 10, caractérisé en ce que l'alcool $R^5$-OH et le méthanol, l'éthanol, le n-propanol, l'i-propanol ou le cyclohexanol.

**12.** Procédé pour la préparation de composés de formule I ou de leurs sels comme ils sont définis dans la revendication 1 ou 2, caractérisé en ce qu'on fait réagir un composé de formule III :

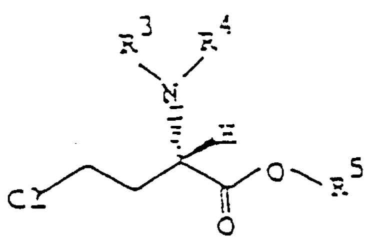

(III)

où

$R^3$, $R^4$ et $R^5$ sont définis comme dans la formule I sauf lorsque $R^5$ est égal à H, avec un composé de formule IV :

$R^1(O)_n$ - $P(OR^2)_2$ (IV)

où

$R^1$, $R^2$ sont définis comme dans la formule I, et dans le cas où on prépare un composé I avec $R^5$ égal à H, on hydrolyse le composé I obtenu initialement

**13.** Procédé selon la revendication 12, caractérisé en ce qu'on réalise la réaction à des températures de 50 à 150 °C.

**14.** Procédé selon la revendication 12 ou 13; caractérisé en ce qu'on réalise la réaction en substance.

**15.** Procédé selon la revendication 14, caractérisé en ce qu'on réalise la réaction à une température de 110 à 140 °C.

**16.** Procédé selon une ou plusieurs des revendications 12 à 15, caractérisé en ce qu'on hydrolyse le composé de formule I obtenu par l'acide chlorhydrique aqueux à 3N à 12N à une température de 90 à 130 °C pour donner le composé de formule I, dans lequel $R^5$ est égal à H.

17. Utilisation de composés de formule III tels qu'ils sont définis dans la revendication 8, pour la préparation de composés de formule I optiquement actifs ou de leurs sels tels qu'ils sont définis dans la formule I selon la revendication 1 ou 2.